(19)

Europäisches
Patentamt

European
Patent Office

Office européen
des brevets

(11)  **EP 2 841 950 B1**

(12)  **EUROPEAN PATENT SPECIFICATION**

(45) Date of publication and mention
of the grant of the patent:
**19.09.2018  Bulletin 2018/38**

(21) Application number: **13719509.5**

(22) Date of filing: **25.04.2013**

(51) Int Cl.:
***G01N 33/68*** (2006.01)

(86) International application number:
**PCT/EP2013/058619**

(87) International publication number:
**WO 2013/160397 (31.10.2013 Gazette 2013/44)**

(54)  **QUANTIFICATION OF IMPURITIES FOR RELEASE TESTING OF PEPTIDE PRODUCTS**

QUANTIFIZIERUNG VON VERUNREINIGUNGEN ZUR FREIGABEPRÜFUNG VON PEPTIDPRODUKTEN

QUANTIFICATION D'IMPURETES POUR TEST D'AUTORISATION DE PRODUITS PEPTIDIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.04.2012  PCT/EP2012/057771**

(43) Date of publication of application:
**04.03.2015  Bulletin 2015/10**

(73) Proprietor: **Sanofi-Aventis Deutschland GmbH 65929 Frankfurt am Main (DE)**

(72) Inventors:
• **VOGEL, Martin
  65926 Frankfurt am Main (DE)**
• **MUELLER, Werner
  65926 Frankfurt am Main (DE)**

(74) Representative: **Weickmann & Weickmann PartmbB
Postfach 860 820
81635 München (DE)**

(56) References cited:
**WO-A2-2012/012352**

• **RYAN D. LEIB ET AL: "Direct Quantitation of Peptide Mixtures without Standards Using Clusters Formed by Electrospray Ionization Mass Spectrometry", ANALYTICAL CHEMISTRY, vol. 81, no. 10, 15 May 2009 (2009-05-15), pages 3965-3972, XP055048311, ISSN: 0003-2700, DOI: 10.1021/ac900294r**
• **GYGI STEVEN P ET AL: "Quantitative analysis of complex protein mixtures using isotope-coded affinity tags", NATURE BIOTECHNOLOGY, vol. 17, no. 10, 1 October 1999 (1999-10-01), pages 994-999, XP002173335, DOI: 10.1038/13690**
• **HONGWEI XIE ET AL: "Characterization of Protein Impurities and Site-Specific Modifications Using Peptide Mapping with Liquid Chromatography and Data Independent Acquisition Mass Spectrometry", ANALYTICAL CHEMISTRY, vol. 81, no. 14, 15 July 2009 (2009-07-15) , pages 5699-5708, XP055048315, ISSN: 0003-2700, DOI: 10.1021/ac900468j**

SEQUENCE LISTING

<110> Sanofi-Aventis Deutschland GmbH

<120> Quantification of Impurities for Release Testing of Peptide Products

<130> 52972PWO

<160> 3

<170> BiSSAP 1.0

<210> 1
<211> 44
<212> PRT
<213> Artificial Sequence

<220>
<221> SOURCE
<222> 1..44
<223> /mol_type="protein"
/note="Lixisenatide-(AVE0010)"
/organism="Artificial Sequence"

<220>
<221> MOD_RES
<222> 44
<223> Lysine is modified with an NH2 group.

<400> 1

```
His Gly Glu Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Ser Lys Lys Lys Lys Lys Lys
        35                  40
```

<210> 2
<211> 45
<212> PRT
<213> Artificial Sequence

<220>
<221> SOURCE
<222> 1..45
<223> /mol_type="protein"
/note="Di-Ser(33)-AVE0010"
/organism="Artificial Sequence"

<220>
<221> MOD_RES
<222> 45
<223> Lysine is modified with an NH2 group.

<400> 2

```
His Gly Glu Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Ser Gly Ala Pro Pro Ser Lys Lys Lys Lys Lys Lys
        35                  40                  45
```

<210> 3
<211> 45
<212> PRT
<213> Artificial Sequence

<220>
<221> SOURCE
<222> 1..45
<223> /mol_type="protein"
/note="Di-Ala(35)-AVE0010"
/organism="Artificial Sequence"

<220>
<221> MOD_RES
<222> 45
<223> Lysine is modified with an NH2 group.

<400> 3

```
        His Gly Glu Gly Thr Phe Thr Ser Asp Leu Ser Lys Gln Met Glu Glu
        1               5                   10                  15
        Glu Ala Val Arg Leu Phe Ile Glu Trp Leu Lys Asn Gly Gly Pro Ser
                    20                  25                  30
        Ser Gly Ala Ala Pro Pro Ser Lys Lys Lys Lys Lys Lys
                    35                  40                  45
```

**Description**

The present invention relates to a method for the quantitative determination of an impurity present in a peptide product, wherein the impurity cannot be separated from the main product and optionally from other impurities. The method particularly involves the use of high resolution mass spectrometry (MS) detection with or without high performance liquid chromatography (HPLC). The method can be used for the investigation of the quality of peptides and proteins, particularly of pharmaceutical peptides and proteins, and formulations thereof.

Using well-known recombinant DNA and chemical solid phase synthesis processes, several proteins and peptides have been synthesized for pharmaceutical use. The production of these proteins and peptides, however, often leads to a multiplicity of undesired synthesis by-products. This is especially the case when they are produced by solid phase synthesis. With an increase in length of the peptide/protein, leading to an increase in the synthesis steps, these by-products may be present in 50 to 70% of the crude product.

Thus, an important part of the synthesis is the purification of the main product from the synthesis by-products. This purification is mainly done by chromatographic procedures. For use as an active ingredient in a pharmaceutical product, the final purified peptide product needs to be analyzed with regard to the purity, which is also done mostly using chromatographically procedures. Since the differences in the molecular structure of the desired peptide product and the undesired synthesis by-products are often very minor compared with the overall structure, the chromatographic properties of these products may be identical or nearly identical. This often leads to a co-elution of these impurities with other impurities and the main product itself in a chromatographic separation procedure.

Leib et al. ("Direct Quantitation of Peptide Mixtures without Standards Using Clusters Formed by Electrospray Ionization Mass Spectrometry", ANALYTICAL CHEMISTRY, vol. 81, no. 10, pp. 3965-3972) relates to a method of direct quantification of peptide mixtures using clusters formed by electrospray ionization mass spectrometry (ESI-MS) without standards for determining impurities. The method is described for five different peptide mixtures with relative concentrations ranging from 0,05% to 50% consisting of leucine/enkephalin, methionine/enkephalin and bradykinin, bradykinin fragment 1-8 and bradykinin fragment 2-9.

The International Patent Application WO 2012/012352A2 discloses modified peptides or analogues thereof, which offer higher binding affinity for its target than the binding affinity of the unmodified peptide. For example, compounds with improved properties compared to enfurvitide useful for the treatment of HIV and AIDS, or compounds with improved properties compared to exenatide (exendin-4) or even lixisenatide, useful for the treatment of diabetes mellitus type 2 in subjects diagnosed with diabetes or for the treatment of pre-diabetic individuals. To manufacture such peptides, various methods of production and purification are disclosed including mass spectrometry. Nevertheless, WO 2012/012352A2 does not provide a method directed to the quantitative determination of impurities.

The development of selective analytical methods is one of the key aspects for the release testing of peptide product compositions used as pharmaceutical compounds. But due to the reasons described above, even great efforts may not result in the separation of all relevant impurities by chromatographic methods. Thus, there is a need to provide novel methods for determining the amounts of impurities in peptide products, particularly the amount of impurities which cannot be quantitatively separated from the desired main product by chromatographic methods.

The present invention provides a method for the quantitative determination of impurities in peptide product compositions, which can not be separated chromatographically from the main product or which co-elute with other impurities or ingredients of the composition. This method is shown exemplarily for the peptide Lixisenatide (AVE0010), a GLP-1 agonist having a length of 44 amino acids long. The amino acid sequence of Lixisenatide is shown in SEQ ID NO:1:

H-G-E-G-T-F-T-S-D-L-S-K-Q-M-E-E-E-A-V-R-L-F-I-E-W-L-K-N-G-G-P-S-S-G-

A-P-P-S-K-K-K-K-K-K-NH$_2$

Lixisenatide is produced by a chemical solid phase synthesis process. By means of chromatographic purification procedures, most of the impurities can be separated from the desired main product.

But nevertheless two impurities, Di-Ser(33)-AVE0010 and Di-Ala(35)-AVE0010, cannot be separated by chromatographic methods. The amino acid sequences of these impurities are as follows:

Di-Ser(33)-AVE0010 (SEQ ID NO:2) H-G-E-G-T-F-T-S-D-L-S-K-Q-M-E-E-E-A-V-R-L-F-I-E-W-L-K-N-G-G-P-S-S-S-G-A-P-P-S-K-K-K-K-K-K-NH$_2$

Di-Ala(35)-AVE0010 (SEQ ID NO:3) H-G-E-G-T-F-T-S-D-L-S-K-Q-M-E-E-E-A-V-R-L-F-I-E-W-L-K-N-G-G-P-S-S-G-
A-A-P-P-S-K-K-K-K-K-K-NH$_2$

Since Lixisenatide is used as a pharmaceutical product, particularly in the treatment of diabetes patients, the presence and the amount of the above impurities has to be determined in order to allow batch release. The present inventors have now found that a quantitative determination of the above impurities may be achieved using high resolution mass spectrometric techniques with or without prior chromatography.

A subject matter of the present invention is a method for the quantitative determination of an impurity present in a peptide product composition, comprising the steps:

(a) providing a peptide product composition comprising a peptide product, which is an exendin peptide product comprising the amino acid sequence S-S-G-A and an unknown amount of at least one impurity, wherein said impurity, which is a Di-Ser(33)-exendin peptide and/or a Di-Ala(35)-exendin peptide and which differs from the desired peptide product by a dimeric amino acid building block of Di-serine and/or Di-alanine, cannot be separated from the peptide product or another ingredient of the composition by a chromatographic procedure,

(b) providing at least one sample of the peptide product composition without said impurity added and optionally at least one further sample of the composition with a known amount of said impurity added,

(c) quantitatively determining said impurity in said at least one sample from step (b) by mass spectrometry, and

(d) calculating the amount of said impurity in the peptide product composition based on the results of (c).

A further subject matter of the present invention is a method for the quantitative determination of an impurity present in a peptide product composition, comprising the steps:

(a) providing a peptide product composition comprising a peptide product, which is an exendin peptide product comprising the amino acid sequence S-S-G-A and an unknown amount of at least one impurity, wherein said impurity, which is a Di-Ser(33)-exendin peptide and/or a Di-Ala(35)-exendin peptide and which differs from the desired peptide product by a dimeric amino acid building block of Di- serine and/or Di-alanine, cannot be separated from the peptide product or another ingredient of the composition by a chromatographic procedure,

(b) providing at least three samples of the peptide product composition, wherein a first sample comprises the peptide product composition without said impurity added, and wherein at least two further samples comprise the peptide product composition each with a different known amount of said impurity added,

(c) quantitatively determining said impurity in said at least three samples from step (b) by mass spectrometry and

(d) calculating the amount of said impurity in the peptide product composition based on the results of (c).

A further subject matter of the invention is a reagent kit for determining the amount of impurities in a Lixisenatide (AVE 0010) product composition, comprising:

(i) at least one stock preparation of Di-Ser(33)-AVE0010 and/or
(ii) at least one stock preparation of Di-Ala(35)-AVE0010.

Still, a further subject-matter of the invention is a method for the quality control of a composition comprising an exendin peptide product comprising the amino acid sequence S-S-G-A, preferably of a composition comprising a Lixisenatide (AVE0010) product, comprising quantitatively determining the amount of a Di-Ser(33)-peptide, e.g. Di-Ser(33)-AVE0010 and/or a Di-Ala(35)-peptide, e.g. Di-Ala(35)-AVE0010, in said composition.

The present invention relates to the determination of an impurity present in a peptide product composition. The term *"peptide product"* encompasses peptides and proteins having a length of at least 5 or at least 10 amino acids and up to 50 or up to 100 amino acids or even longer. The peptide product may consist of genetically encoded amino acid building blocks or may comprise non-genetically encoded amino acid building blocks, e.g. non-naturally occurring amino acids, D-amino acids or chemically modified amino acids or may consist of several peptide chains linked e.g. by disulfide bridges. The peptide product may further contain modifications at the N- and/or C-terminus and/or at side chains, e.g. an acylation, an amidation or the addition of non-peptide side chain groups such as lipophilic groups. The peptide product may be linear or circular. The peptide product has a length from 5-100 amino acids.

The synthesis of the peptide product may involve recombinant DNA processes and/or chemical synthesis processes. Preferably, the peptide product has been chemically synthesized, particularly by a solid phase synthesis procedure which is well-known in the art, e.g. a procedure involving a stepwise elongation of a peptide chain coupled to a carrier, e.g. a synthetic resin. In a preferred embodiment of the invention, the peptide product is an Exendin peptide, e.g. Exendin-4, Liraglutide or Lixisenatide (AVE0010). More preferably, the peptide product is an Exendin peptide, e.g. an Exendin peptide comprising the amino acid sequence S-S-G-A, most preferably Lixisenatide (AVE0010).

The method of the present invention involves the quantitative determination of an impurity present in a peptide product after its synthesis, e.g. after its synthesis by a solid phase synthesis procedure or as a degradation product after storage. The impurity is usually a peptide impurity, e.g. a peptide which differs from the desired main peptide product in at least one amino acid building block and/or a chemical modification. For example, the impurity may be a peptide which differs from the desired peptide product by at least one dimeric amino acid building block, i.e. a Di-serine or Di-alanine building block. In the case of an Exendin peptide, e.g. an Exendin peptide comprising the amino acid sequence S-S-G-A, the impurity may be e.g. a Di-Ser(33)-exendin peptide, a Di-Ala(35)-exendin peptide or combinations thereof. In the case of Lixisenatide, the impurity may e.g. be Di-Ser(33)-AVE0010, Di-Ala(35)-AVE0010 or combinations thereof.

The method of the present invention comprises the quantitative determination of at least one impurity in a peptide product composition. The peptide product composition comprises at least one peptide product and at least one impurity to be determined. Further, the composition may comprise other ingredients, e.g. peptide or non-peptide ingredients including other impurities. The peptide product composition may e.g. be a pharmaceutical formulation or a composition intended for the manufacture of a pharmaceutical formulation, e.g. a synthesized batch of the peptide product.

In the method of the present invention, the peptide product comprises an unknown amount of at least one impurity, which cannot be quantitatively separated from the peptide product by a chromatographic procedure or which co-elutes with other impurities or ingredients present in the peptide product sample. In some cases, the peptide product composition comprises unknown amounts of one impurity. In other cases, the peptide product composition comprises unknown amounts of at least two, e.g. 2, 3, 4, 5 or even more impurities which cannot be quantitatively separated from the peptide product by a chromatographic procedure or which co-elute with other other impurities or ingredients present in the peptide product sample. Preferably, the at least one impurity cannot be quantitatively separated from the peptide product.

The term *"chromatographic procedure"* involves a chromatographic procedure suitably for the purification of peptide products, including e.g. ion exchange chromatography, hydrophobic interaction chromatography, affinity chromatography, size exclusion chromatography, and particularly high performance liquid chromatography (HPLC) and more particularly Reverse Phase HPLC, or combinations of several procedures.

The impurity which is determined by the method of the present invention cannot be quantitatively separated from the peptide product or from other ingredients of the composition. This means, the impurity co-elutes or substantially co-elutes with the desired peptide product or with other impurities or ingredients after a chromatographic separation procedure, particularly after a chromatographic separation procedure as described above in such a way that quantification by optical methods, e.g UV- or fluorescence detection is not possible. This means, that the impurity cannot be separated from the desired peptide product or from other ingredients including impurities by reasonable efforts and its quantitative amount in a given batch of the peptide product composition has to be quantitatively determined by the method of the present invention.

Step (a) of the inventive method comprises providing a peptide product composition comprising an unknown amount of at least one impurity wherein that impurity cannot be separated from the peptide product or another ingredient of the composition by a chromatographic procedure as described above. The peptide product may e.g. be a product for use in pharmaceutical applications, e.g. for use as a medicament in human or veterinary medicine.

Step (b) of the inventive method involves providing at least one sample of the peptide product composition without added impurity and at least one further sample of the peptide product composition with a known amount of added impurity. In a preferred embodiment, step (b) involves providing at least three samples of the peptide product composition wherein the first sample comprises the peptide product composition without added impurity in wherein at least two further samples comprise the peptide product composition and each of these samples additionally comprises a different known amount of added impurity.

At least one sample is a sample which comprises the peptide product composition without added impurity, i.e. an un-spiked sample comprising an unknown amount of the impurity to be determined. In addition to this sample, at least one, e.g. at least two, three or four further samples may be provided which are spiked samples comprising the peptide product composition with the unknown amount of impurity and additionally with different known amounts of added impurity to be determined. These spiked samples may be prepared by adding the impurity to the un-spiked peptide product composition samples from a stock preparation of the impurity, preferably from at least two stock preparations comprising different concentrations of the impurity. The stock preparations may be present in any suitable form, e.g. preparations in dry or liquid form. Preferably the stock preparations are stock solutions.

The tested samples comprise the peptide product with an unknown amount of the impurity to be determined and the added impurity. These compounds are present in concentrations, which allow analysis by mass spectrometry. The concentration of the peptide product may vary to a great extent. It may be e.g. in the range of 0.001 - 50 mg/ml, preferably 0.01-10 mg/ml and more, preferably 0.02 - 5.0 mg/ml, for example about 1.0 mg/ml. In the spiked samples, the amount of added impurity is preferably in the range of 0.1%-5%, preferably from 0.1-2% based on the concentration of the peptide product in the sample.

In a preferred embodiment, the method of the invention comprises the testing of an un-spiked peptide product sample together with at least one, preferably at least two, three or four or even more peptide product samples spiked with at least one impurity. In a different embodiment, the method of the invention involves the testing of only an un-spiked peptide product sample. The unknown amount of impurity present in the un-spiked sample may be determined by reference measurements of spiked samples and/or by comparing the measured value of the impurity in the un-spiked sample with a reference, e.g. a standard or calibration curve.

Step (c) of the inventive method comprises quantitatively determining said impurity in said at least one sample from step (b) by high resolution mass spectrometry. In addition to mass spectrometry, the determination may involve a prior chromatographic procedure, e.g. in order to separate other impurities from the peptide product or from other ingredients of the composition. Preferably, mass spectrometry is combined with HPLC. Each sample may subjected to several individual determinations in order to increase the accuracy of the measurement.

Mass spectrometry is based on a measurement of the mass-to-charge ratio of charged particles. In a typical mass spectrometry procedure, the sample is loaded onto the mass spectrometry instrument and volatilized. The sample components are ionized and the resulting ions are separated in the mass analyzer by electromagnetic fields. The resulting ions are detected and the signal is processed into a mass spectrum. For the ionization of peptide products, electrospray ionization (ESI) and matrix-assisted laser desorption/ ionization (MALDI) may be used. The resulting ions may be detected by highly sensitive methods such as Orbitrap or Fourier Transform (FT)-Ion Cyclotron Resonance (ICR) detection systems.

According to step (c), at least one peak associated with the impurity to be determined is analyzed by mass spectrometry. This peak may be derived either from a deconvoluted or non-deconvoluted mass spectrum. Preferably, the method involves the determination of one or several monoisotopic peaks associated with the impurity to be determined. In the case of the impurity Di-Ser(33)-AVE0010, a quadruply charged peak of a non-deconvoluted mass spectrum at e.g.1237.1529 Da or of the deconvoluted mass spectrum at 4944.5822 Da may be used. In case of the impurity Di-Ala(35)-AVE0010, a quadruply charged peak of the non-deconvoluted mass spectrum at 1233.1550 Da or of the deconvoluted mass spectrum at 4928.5908 Da may be analyzed.

The at least one peak to be analyzed may be identified in a mass spectrum of the impurity using a high resolution setting and a highly sensitive detection as described above.

Step (d) comprises calculating the amount of the impurity in the peptide product composition based on the mass spectrometry analysis results. In a preferred embodiment, the calculation is carried out by a method comprising a regression analysis, particularly a linear regression analysis. The regression analysis may comprise generating a curve, e.g. a straight line, from the measured values of the impurity in at least two spiked samples, i.e. samples comprising the peptide product with the unknown amount of impurity and having added the two different known amounts of impurities (or from reference values of such samples which have been generated in standard or calibration measurements). Into this curve, e.g. a straight line, the measured value of the impurity in the un-spiked sample is inserted thereby allowing quantitative determination of the unknown amount of the impurity present in that sample.

Preferably, the calculation is carried out according to the linear straight line equation:

$$y = ax + b$$

wherein y corresponds to the determined peak area of the impurity in a sample (either spiked or un-spiked sample), x corresponds to the known added amount of the impurity in a spiked sample, a is the straight line slope and b is the intercept with the y-axis, which corresponds to the measurement signal of the impurity in a sample without added impurity (x = 0). The quantitative amount of impurity in the un-spiked sample $x_t$ may be obtained using the obtained regression parameters as follows:

$$x_t = b \cdot a^{-1}$$

The invention also encompasses a reagent kit for determining the amount of impurities in a Lixisenatide product which comprises at least one stock preparation of Di-Ser(33)-AVE0010 and/or Di-Ala(35)-AVE0010 and optionally further reagents such as solvents, buffers etc. The reagent kit may be used in a method for the quality control of a Lixisenatide product for the quantitative determination of the amount of Di-Ser(33)-AVE0010 and/or Di-Ala(35)-AVE0010 in said Lixisenatide product. Preferably, the quantitative determination is carried according to a mass spectrometric method, e.g. a mass spectrometric method as described above.

The present invention is explained in more detail by the following Figures and Examples.

Legends of figures:

Figure 1 shows the co-elution of the peaks of Lixisenatide (AVE0010), Di-Ser(33)-AVE0010 and Di-Ala(35)-AVE0010 in an HPLC chromatogram.

Figure 2 shows the mass spectrum of the quadruply charged molecular ions of AVE0010 and the impurities Di-Ser(33)-AVE0010 and Di-Ala(35)-AVE0010.

Figure 3 shows a comparison of mass spectra of un-spiked and spiked (1% of each impurity) AVE0010 preparations.

Figure 4 shows an expanded view of the mass range of Di-Ser(33)-AVE0010 at the resolution setting of 60,000 and 100,000 respectively at m/z400 and the theoretical calculated pattern of overlapping species.

Figure 5 shows deconvoluted mass spectra with theoretical calculated patterns of overlapping species.

Figure 6 shows an expanded view on the impurity peak used for the quantification of Di-Ser(33)-AVE0010.

Figure 7 shows an extracted single ion chromatogram of a quadruply charged isotope of Di-Ala(35)-AVE0010 and Di-Ser(33)-AV0010 in a test sample as a non-deconvoluted mass spectrum.

Figure 8 shows an extracted single ion chromatogram of Di-Ala(35)-AVE0010 and Di-Ser(33)-AV0010 in a test sample as a deconvoluted mass spectrum.

Figure 9 shows an example for calculation of the concentration of the impurity Di-Ala(35)-AVE0010 in a test sample.

Examples

**Analytical Procedure for Quantification of Di-Ser(33)-Lixisenatide and Di-Ala(35)-Lixisenatide by HPLC/MS**

1.Materials and Methods

1.1 Reference materials

Lixisenatide (AVE0010) Reference material; produced by solid phase synthesis, followed by purification.
Di-Ser(33)-AVE0010 Reference material; produced by solid phase synthesis process with double coupling steps of the protected amino acid Serine at position 33, followed by purification.
Di-Ala(35)-AVE0010 Reference material; produced by solid phase synthesis process with double coupling steps of the protected amino acid Alanine at position 35, followed by purification.
An un-spiked sample preparation of Lixisenatide (containing unknown amounts of impurities DiSer(33)-AVE0010 and DiAla(35)-AVE0010) was prepared at a concentration of 1.0 mg Lixisenatide/ml.
Four spiked sample preparations for each of the impurities were prepared by incorporating 0.2%, 0.4%, 0.6% and 0.8% by weight of the impurities Di-Ser(33)-AVE0010 or Di-Ala(35)-AVE0010, respectively, in a sample preparation with 1.0 mg Lixisenatide/ml.

1.2 Analytical conditions

A gradient high performance liquid chromatographic system consisting of a binary HPLC pump, an autosampler, column oven, and a high resolution mass spectrometer e.g. LTQ-Orbitrap (ThermoFisher) with a resolution setting of 100.000 (at m/z 400), or equivalent was used.
Chromatography was performed with a C18 Reversed Phase analytical HPLC column (Jupiter C18 3$\mu$m 300A) with a length of 150 mm and an internal diameter of 2.0 mm.
Mobile Phase A consisted of 850 volume parts water, 150 volume parts acetonitrile and 1 volume part trifluoroacetic acid. Mobile Phase B consisted of 250 volume parts water, 750 volume parts acetonitrile and one volume part trifluoroacetic acid. The gradient was set as follows:

| Time [minutes] | Mobile phase A [%] | Mobile Phase B [%] |
|---|---|---|
| 0.00 | 77.5 | 22.5 |
| 17.50 | 28.0 | 72.0 |
| 17.51 | 77.5 | 22.5 |
| 26.00 | 77.5 | 22.5 |

The mass spectrometer was operated in positive electrospray ionization mode and scanned in a way to measure the ion signals from the analytes to determined.

The test conditions were as follows:

| | |
|---|---|
| Flow rate: | 0.25 mL/min |
| Injection volume: | 10 μL |
| Autosampler temperature: | Set autosampler temperature at +10°C±2°C |
| Column temperature: | Set oven temperature at +25°C±2°C |
| Ionization method: | ESI positive |

Measurement/Detection:

| | | |
|---|---|---|
| ITMS: | mode: | positive |
| | mass range: | 700.0-2000.0 |
| FTMS: | mode: | positive |
| | mass range(SIM) | 1232.0-1239.0 |
| | resolution: | 100,000 |
| Divert Valve: | waste | 0 - 1.5 min |
| | inject | 1.5 2.25.5 min |
| PDA total scan (UV), optional | | |

The procedure was based on the methods described in Ph. Eur 7.0 (2011) (2.2.43 Mass Spectrometry) and USP 34 (2011) (<736> Mass Spectrometry).

1.3 Integration

Determinations were performed either from the deconvoluted mass spectra or the non-deconvoluted mass spectra of the quadruply charged ions $[M+4H^+]^{4+}$.

After selection of the accurate mass of a single isotopic peak of the pattern:

a) using a quadruply charged peak of the non deconvoluted mass spectra:

- DiSer(33)-AVE0010: e.g. 1237.1529 Da
- DiAla(35)-AVE0010: e.g. 1233.1550 Da,
  or b) using the deconvoluted mass spectra:
- DiSer(33)-AVE0010: e.g. 4944.5822 Da
- DiAla(35)-A VE0010: e.g. 4928.5908 Da,

extracted ion chromatograms were performed. Only the isotope of interest was extracted. The peaks from the extracted ion chromatograms were integrated.

2.Results

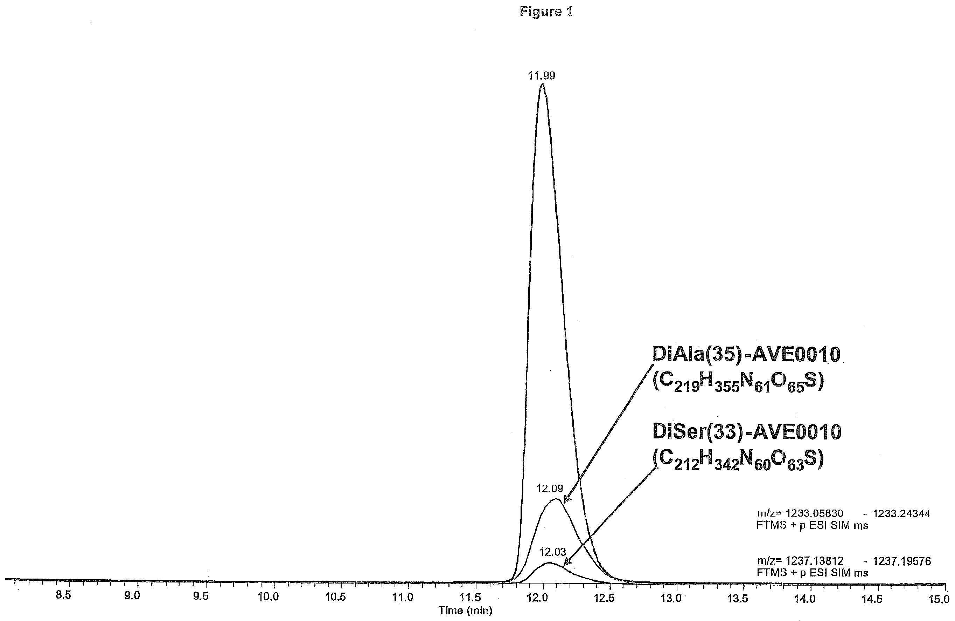

Figure 1 shows the co-elution of the peaks for Lixisenatide and the impurities Di-Ala(35)-AVE0010 and Di-Ser(33)-AVE0010 in an HPLC chromatogram. Due to these overlaps in the chromatographic peaks, Di-Ala(35)-AVE0010 and Di-Ser(33)-AVE0010 constitute impurities which cannot be quantitatively separated from the desired peptide product AVE0010 by a chromatographic procedure.

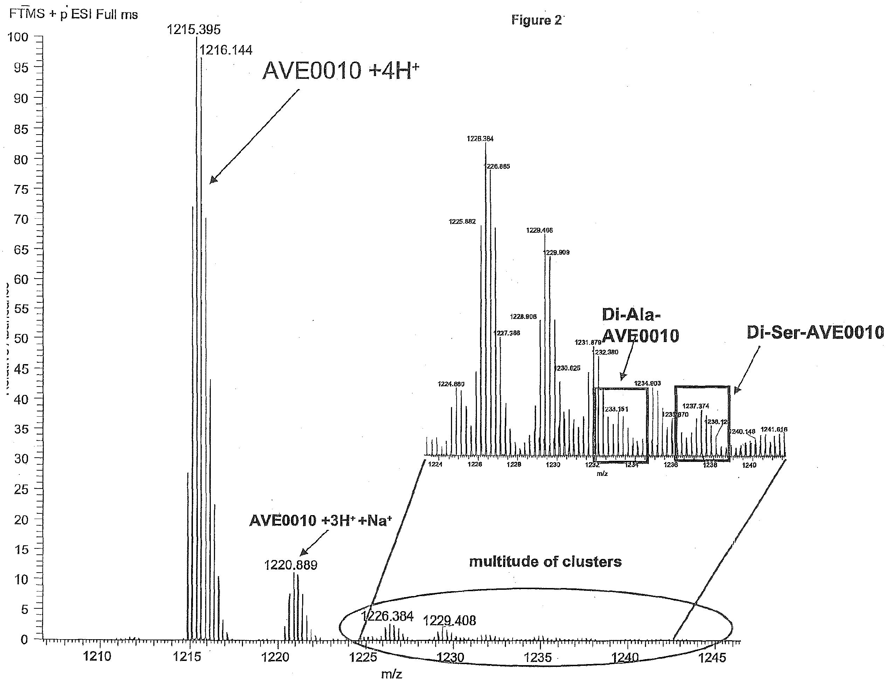

For the method development some specific features of mass spectrometry had to be taken into account. The mass spectrum of Lixisenatide shows besides the multiply protonated species typically clustering with cations that are ubiquitous in solution, e.g. sodium or potassium. Figure 2 shows the spectrum section of the quadruply charged ions of AVE0010 which overlap with the mass spectra of the impurities and thereby obscure the analyte.

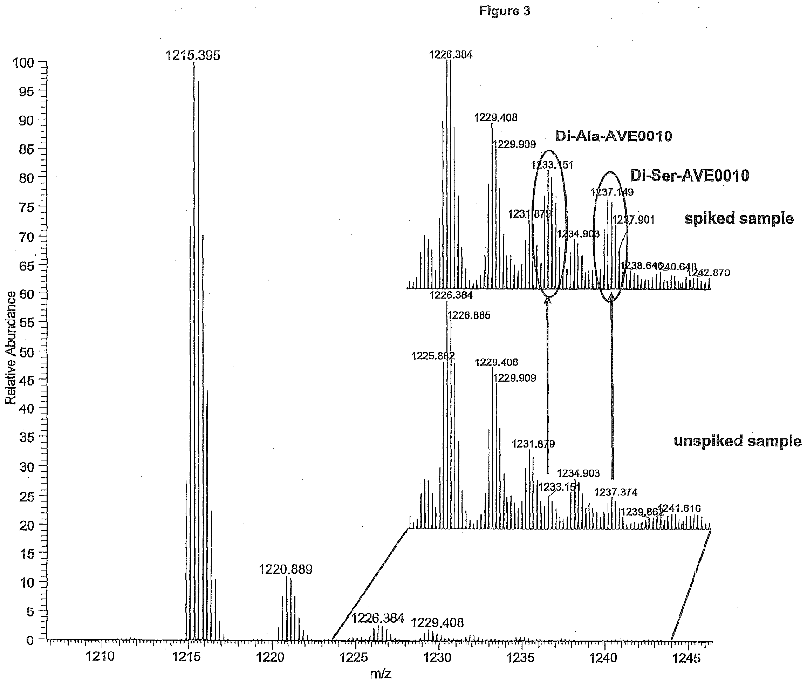

In Figure 3 an AVE0010 sample spiked with 1% of the two impurities is compared with an un-spiked sample containing yet a low amount of the impurities. In the expanded view of the inset can be seen, that there may be an overlap of the molecular patterns with underlying clusters. In the case of Di-Ser(33)-AVE0010 the mass increase relative to Lixisenatide is 87 Da. On the other hand, Lixisenatide can cluster with 4 sodium ions leading to a mass shift of 88 Da. These two patterns overlap and have to be separated mass-spectrometrically otherwise the Di-Ser-AV0010 impurity cannot be quantified specifically.

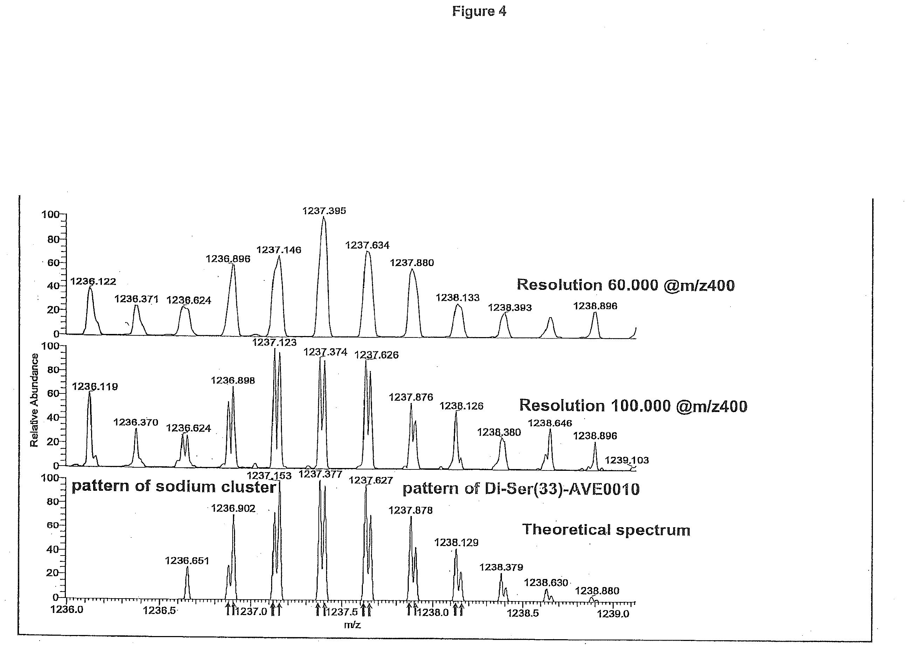

As can be seen in Figure 4, the overlapping peaks were not separated with a resolution setting of 60.000 but only with a resolution setting of 100.000. In the lower part of Figure 4 the theoretical spectrum of Di-Ser-AVE0010 overlapping with the sodium cluster of Lixisenatide is shown. Such high resolutions are preferably delivered by Fourier transform mass spectrometry, either FT-ICR or as used here by an FT-Orbitrap mass spectrometer.

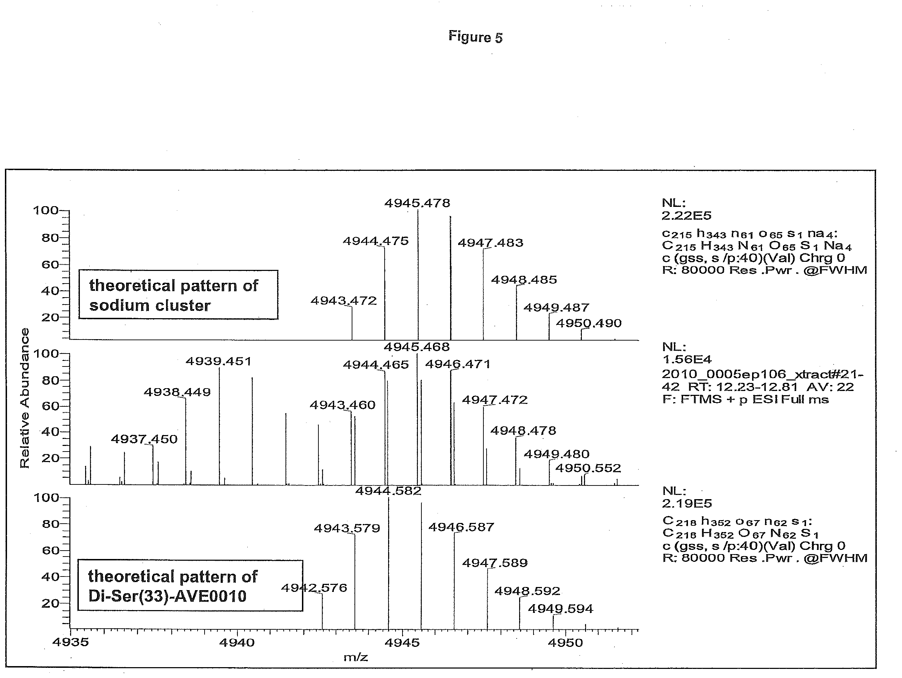

After deconvolution and centroiding of the spectra, these two species may be separated mass-spectroscopically, c.f. Fig.5.

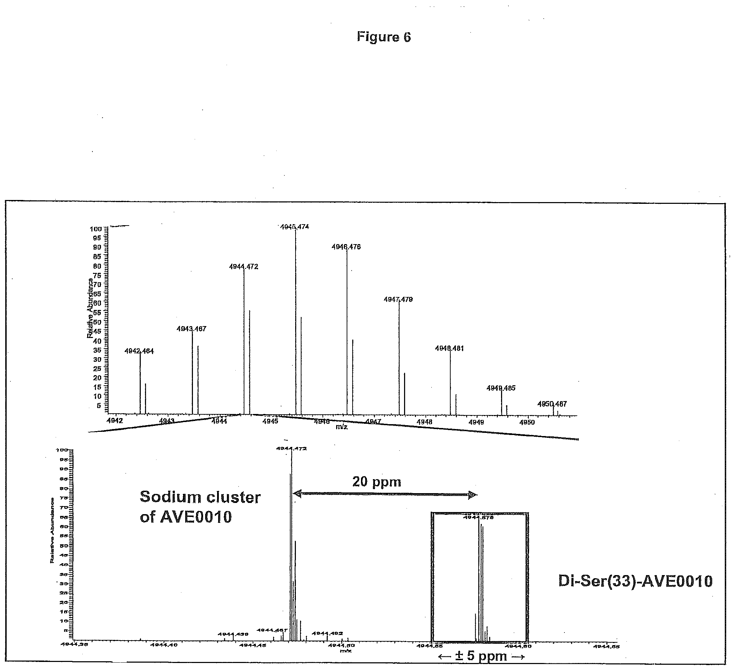

In Figure 6 all measured individual mass spectra of the complete chromatographic peak have been added. The mass peak used for the measurement of Di-Ser(33)-AVE0010 is shown in an expanded view. It is sufficiently distant from the peak corresponding to the sodium cluster of AVE0010 to allow an accurate measurement. All analyte mass peaks fall within a narrow mass window, showing the mass stability of the instrument, well separated from any interfering peak. Therefore this method is suited to determine specifically the amounts of Di-Ser(33)-AVE0010 and Di-Ala(35)-AVE0010 in a Lixisenatide peptide product.

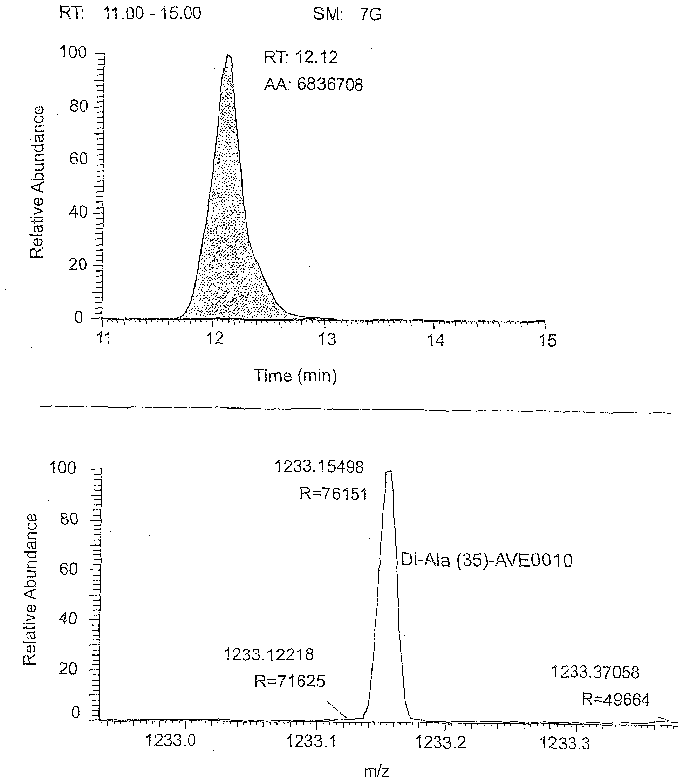

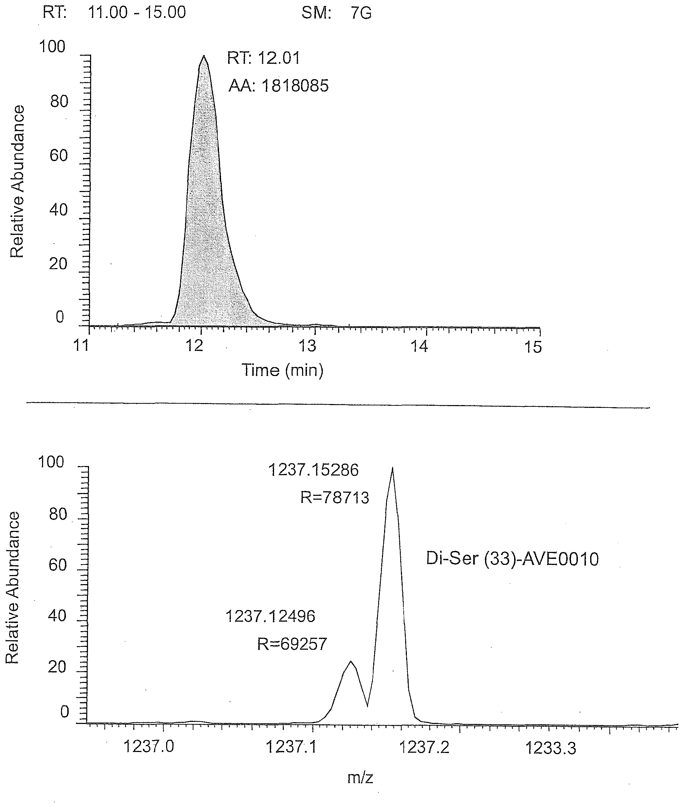

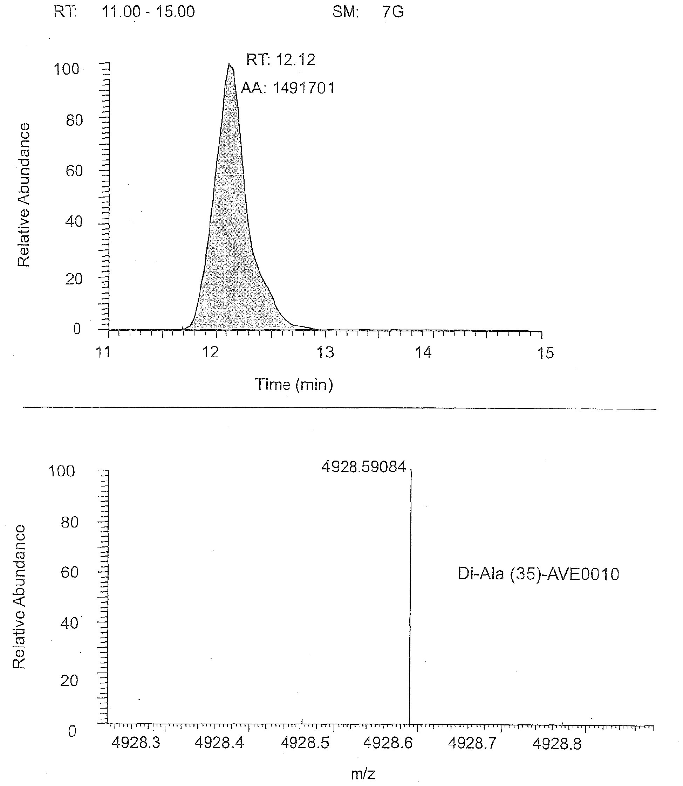

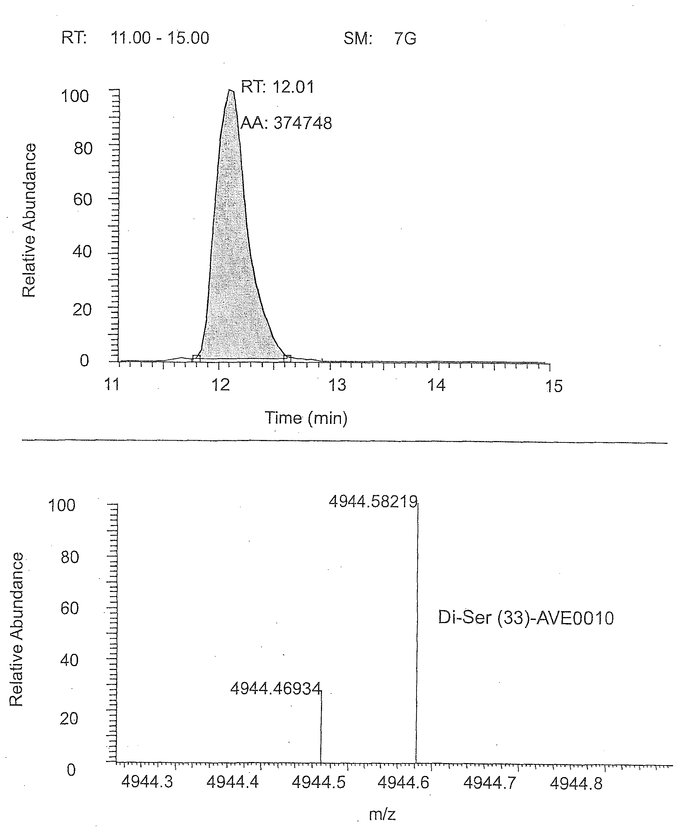

The extracted ion chromatograms of a quadruply charged isotope of Di Ala(35)-AVE0010 and Di-Ser(33)-AV0010 in a test sample are shown as non-deconvoluted mass spectra in Figure 7 and as deconvoluted mass spectra in Figure 8.

The amount of Di-Ser(33)-AVE0010 and Di-Ala(35)-AVE0010 may be calculated by a linear regression analysis. By using each injection of the spiked and un-spiked test solutions, a regression curve may be established according to the equation:

$$y = ax+b$$

a = Slope
y = Peak area of test solutions (un-spiked and spiked test solutions)
x = Added amount of the Di-Ser(33)-AVE0010 or Di-Ala(35)-AVE0010 in test solutions (unspiked and spiked test solutions).
b = Intercept

The concentration $x_t$ of Di-Ser(33)-AVE0010 and Di-Ala(35)-AVE0010 in the test sample may be calculated using the regression parameters obtained as follows:

$$x_t = b \cdot a^{-1}$$

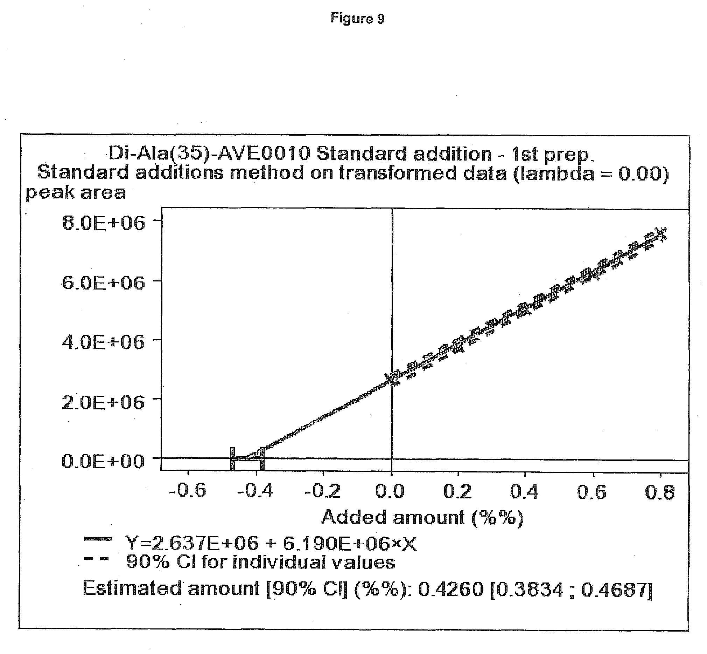

Figure 9 shows an example for calculation of the concentration Di-Ala(35)-AVE0010 in the test sample.

The final result was determined as the arithmetic mean of all individual determinations expressed in percent of Lixisenatide. In the specific case, the mean value of the impurity Di-Ala(35)-AVE0010 in the sample was determined as 0.4260% based on the amount of Lixisenatide.

## Claims

1. A method for the quantitative determination of an impurity present in a peptide product composition, comprising the steps:

   (a) providing a peptide product composition comprising a peptide product, which is an exendin peptide product comprising the amino acid sequence S-S-G-A and an unknown amount of at least one impurity which is a Di-Ser(33)-exendin peptide and/or a Di-Ala(35)-exendin peptide and which differs from the desired peptide product by a dimeric amino acid building block of Di-serine and/or Di-alanine, wherein said impurity cannot be separated from the peptide product or another ingredient of the composition by a chromatographic procedure and wherein said impurity co-elutes or substantially co-elutes with the desired peptide product after the chromatographic separation procedure,
   (b) providing at least one sample of the peptide product composition without said impurity added and at least one further sample of the peptide product composition with a known amount of said impurity added,
   (c) quantitatively determining said impurity in said sample from step (b) by mass spectrometry, and
   (d) calculating the amount of said impurity in the peptide product composition based on the results of (c).

2. A method for the quantitative determination of an impurity present in a peptide product composition, comprising the steps:

   (a) providing a peptide product composition comprising a peptide product , which is an exendin peptide product comprising the amino acid sequence S-S-G-A and an unknown amount of at least one impurity which is a Di-Ser(33)-exendin peptide and/or a Di-Ala(35)-exendin peptide and which differs from the desired peptide product by a dimeric amino acid building block of Di-serine and/or Di-alanine, wherein said impurity cannot be separated from the peptide product or another ingredient of the composition by a chromatographic procedure,
   (b) providing at least three samples of the peptide product composition, wherein a first sample comprises the peptide product composition without said impurity added, and wherein at least two further samples comprise the peptide product composition each with a different known amount of said impurity added,
   (c) quantitatively determining said impurity in said samples from step (b) by mass spectrometry, and
   (d) calculating the amount of said impurity in the peptide product composition based on the results of (c).

3. The method of claim 1 or 2, wherein the peptide product has been chemically synthesized, particularly by a solid phase synthesis procedure, or produced by recombinant DNA processes.

4. The method of any one of claims 1-3, wherein the peptide product composition is a pharmaceutical formulation or a composition intended for the manufacture of a pharmaceutical formulation.

5. The method of any one of claims 1-4, wherein the exendin peptide product is Lixisenatide (AVE0010).

6. The method of any one of claims 1-5, wherein the peptide product comprises unknown amounts of at least 2 impurities which cannot be quantitatively separated from the peptide product or from another ingredient of the composition by a chromatographic procedure.

7. The method of any of claims 1-6, wherein the impurity is added to at least one peptide product composition sample from a stock preparation, preferably from at least two stock preparations comprising different concentrations of the impurity.

8. The method of any one of claims 1-7, wherein at least 3 or 4 further samples with different known amounts added impurity are provided and subjected to mass spectrometry determination.

9. The method of any one of claims 1-8, wherein the mass spectrometry is high resolution mass spectrometry, e.g. comprising Fourier Transform mass spectrometry.

10. The method of any one of claims 1-9, wherein the calculation comprises a linear regression analysis.

11. The method of claim 10, wherein the calculation is carried out according to the equation:

$$y = ax + b$$

wherein

a = slope
y = determined peak area of the impurity in a sample
x = added amount of the impurity in a sample
b = intercept

and the unknown amount of the impurity $x_t$ is obtained as follows:

$$x_t = b \cdot a^{-1}$$

12. A reagent kit for determining the amount of impurities in a Lixisenatide (AVE 0010) product composition, comprising:

(i) at least one stock preparation of Di-Ser(33)-AVE0010 (SEQ ID NO:2) H-G-E-G-T-F-T-S-D-L-S-K-Q-M-E-E-E-A-V-R-L-F-I-E-W-L-K-N-G-G-P-S-S-S-G-A-P-
P-S-K-K-K-K-K-K-NH$_2$

and/or
(ii) at least one stock preparation of Di-Ala(35)-AVE0010 (SEQ ID NO:3) H-G-E-G-T-F-T-S-D-L-S-K-Q-M-E-E-E-A-V-R-L-F-I-E-W-L-K-N-G-G-P-S-S-G-A-A-P-
P-S-K-K-K-K-K-K-NH$_2$.

13. A method for the quality control of a composition comprising an exendin peptide product comprising the amino acid sequence S-S-G-A, comprising quantitatively determining according to the method of any one of claims 1-11 the amount of at least one impurity which is a Di-Ser(33)-exendin peptide and/or a Di-Ala(35)-exendin peptide and which differs from the desired peptide product by a dimeric amino acid building block of Di-serine and/or Di-alanine in said composition.

14. A method for the quality control of a composition comprising a Lixisenatide (AVE0010) product, comprising quantitatively determining according to the method of any one of claims 1-11 the amount of at least one impurity which is a Di-Ser(33)-AVE0010 and/or a Di-Ala(35)-AVE0010 and which differs from the desired peptide product by a dimeric amino acid building block of Di-serine and/or Di-alanine in said composition.

**Patentansprüche**

1. Verfahren zur quantitativen Bestimmung einer Verunreinigung, die in einer Peptidproduktzusammensetzung vorhanden ist, umfassend die folgenden Schritte:

(a) Bereitstellen einer Peptidproduktzusammensetzung, umfassend ein Peptidprodukt, bei dem es sich um ein Exendinpeptidprodukt handelt, das die Aminosäuresequenz S-S-G-A umfasst, und eine unbekannte Menge mindestens einer Verunreinigung, bei der es sich um ein Di-Ser(33)-Exendinpeptid und/oder ein Di-Ala(35)-Exendinpeptid handelt und die sich von dem gewünschten Peptidprodukt durch einen dimeren Aminosäurebaustein aus Di-Serin und/oder Di-Alanin unterscheidet, wobei die Verunreinigung nicht von dem Peptidprodukt oder einem anderen Bestandteil der Zusammensetzung durch ein chromatographisches Verfahren abgetrennt werden kann und wobei die Verunreinigung nach dem chromatographischen Trennverfahren mit dem gewünschten Peptidprodukt coeluiert oder im Wesentlichen coeluiert,
(b) Bereitstellen mindestens einer Probe der Peptidproduktzusammensetzung, zu der nicht die Verunreinigung zugegeben ist, und mindestens einer weiteren Probe der Peptidproduktzusammensetzung, zu der eine bekannte Menge der Verunreinigung zugegeben ist,
(c) quantitatives Bestimmen der Verunreinigung in der Probe aus Schritt (b) mittels Massenspektrometrie und
(d) Berechnen der Menge der Verunreinigung in der Peptidproduktzusammensetzung auf der Basis der Ergebnisse von (c).

2. Verfahren zur quantitativen Bestimmung einer Verunreinigung, die in einer Peptidproduktzusammensetzung vorhanden ist, umfassend die folgenden Schritte:

(a) Bereitstellen einer Peptidproduktzusammensetzung, umfassend ein Peptidprodukt, bei dem es sich um ein Exendinpeptidprodukt handelt, das die Aminosäuresequenz S-S-G-A umfasst, und eine unbekannte Menge mindestens einer Verunreinigung, bei der es sich um ein Di-Ser(33)-Exendinpeptid und/oder ein Di-Ala(35)-Exendinpeptid handelt und die sich von dem gewünschten Peptidprodukt durch einen dimeren Aminosäurebaustein aus Di-Serin und/oder Di-Alanin unterscheidet, wobei die Verunreinigung nicht von dem Peptidprodukt oder einem anderen Bestandteil der Zusammensetzung durch ein chromatographisches Verfahren abgetrennt werden kann,
(b) Bereitstellen von mindestens drei Proben der Peptidproduktzusammensetzung, wobei eine erste Probe die Peptidproduktzusammensetzung umfasst, zu der nicht die Verunreinigung zugegeben ist, und wobei mindestens zwei weitere Proben die Peptidproduktzusammensetzung umfassen, zu der jeweils eine andere bekannte Menge der Verunreinigung zugegeben ist,
(c) quantitatives Bestimmen der Verunreinigung in den Proben von Schritt (b) mittels Massenspektrometrie und
(d) Berechnen der Menge der Verunreinigung in der Peptidproduktzusammensetzung auf der Basis der Ergebnisse von (c).

3. Verfahren nach Anspruch 1 oder 2, wobei das Peptidprodukt chemisch synthetisiert, insbesondere durch ein Festphasensyntheseverfahren, oder durch DNA-Rekombinationsverfahren hergestellt ist.

4. Verfahren nach einem der Ansprüche 1-3, wobei die Peptidproduktzusammensetzung eine pharmazeutische Formulierung oder eine für die Herstellung einer pharmazeutischen Formulierung bestimmte Zusammensetzung ist.

5. Verfahren nach einem der Ansprüche 1-4, wobei es sich bei dem Exendinpeptidprodukt um Lixisenatid (AVE0010) handelt.

6.  Verfahren nach einem der Ansprüche 1-5, wobei das Peptidprodukt unbekannte Mengen von mindestens 2 Verunreinigungen umfasst, die durch ein chromatographisches Verfahren nicht quantitativ von dem Peptidprodukt oder von einem anderen Bestandteil der Zusammensetzung abgetrennt werden können.

7.  Verfahren nach einem der Ansprüche 1-6, wobei die Verunreinigung zu mindestens einer Peptidproduktzusammensetzungsprobe aus einer Stammzubereitung, vorzugsweise aus mindestens zwei Stammzubereitungen, die unterschiedliche Konzentrationen der Verunreinigung umfassen, zugegeben wird.

8.  Verfahren nach einem der Ansprüche 1-7, wobei mindestens 3 oder 4 weitere Proben mit unterschiedlichen bekannten Mengen an zugegebener Verunreinigung bereitgestellt werden und einer Massenspektrometriebestimmung unterzogen werden.

9.  Verfahren nach einem der Ansprüche 1-8, wobei es sich bei der Massenspektrometrie um hochauflösende Massenspektrometrie, die z. B. Fourier-Transform-Massenspektrometrie umfasst, handelt.

10. Verfahren nach einem der Ansprüche 1-9, wobei die Berechnung eine lineare Regressionsanalyse umfasst.

11. Verfahren nach Anspruch 10, wobei die Berechnung gemäß der folgenden Gleichung durchgeführt wird:

$$y = ax+b,$$

wobei

    a = Steigung
    y = bestimmte Peakfläche der Verunreinigung in einer Probe
    x = hinzugefügte Menge der Verunreinigung in einer Probe
    b = Achsenabschnitt

und die unbekannte Menge der Verunreinigung $x_t$ wie folgt erhalten wird:

$$x_t = b \bullet a^{-1}$$

12. Reagenzienkit für die Bestimmung der Menge an Verunreinigungen in einer Lixisenatid(AVE0010)-Produktzusammensetzung, umfassend:

    (i) mindestens eine Stammzubereitung von Di-Ser(33)-AVE0010 (SEQ ID NO: 2) H-G-E-G-T-F-T-S-D-L-S-K-Q-M-E-E-E-A-V-R-L-F-I-E-W-L-K-N-G-G-P-S-S-S-G-A-P-
    P-S-K-K-K-K-K-K-NH$_2$

    und/oder
    (ii) mindestens eine Stammzubereitung von Di-Ala(35)-AVE0010 (SEQ ID NO: 3) H-G-E-G-T-F-T-S-D-L-S-K-Q-M-E-E-E-A-V-R-L-F-I-E-W-L-K-N-G-G-P-S-S-G-A-A-P-
    P-S-K-K-K-K-K-K-NH$_2$.

13. Verfahren zur Qualitätskontrolle einer Zusammensetzung, umfassend ein Exendinpeptidprodukt, das die Aminosäuresequenz S-S-G-A umfasst, umfassend das quantitative Bestimmen gemäß dem Verfahren nach einem der Ansprüche 1-11 der Menge mindestens einer Verunreinigung, bei der es sich um ein Di-Ser(33)-Exendinpeptid und/oder ein Di-Ala(35)-Exendinpeptid handelt und die sich von dem gewünschten Peptidprodukt durch einen dimeren Aminosäurebaustein aus Di-Serin und/oder Di-Alanin unterscheidet, in der Zusammensetzung.

14. Verfahren zur Qualitätskontrolle einer Zusammensetzung, die ein Lixisenatid(AVE0010)-Produkt umfasst, umfassend das quantitative Bestimmen gemäß dem Verfahren nach einem der Ansprüche 1-11 der Menge mindestens einer Verunreinigung, bei der es sich um ein Di-Ser(33)-AVE0010 und/oder ein Di-Ala(35)-AVE0010 handelt und die sich von dem gewünschten Peptidprodukt durch einen dimeren Aminosäurebaustein aus Di-Serin und/oder Di-Alanin unterscheidet, in der Zusammensetzung.

## Revendications

1. Procédé de détermination quantitative d'une impureté présente dans une composition de produit peptidique, comprenant les étapes de :

    (a) fourniture d'une composition de produit peptidique comprenant un produit peptidique, qui est un produit peptidique d'exendine comprenant la séquence d'acides aminés S-S-G-A et une quantité inconnue d'au moins une impureté qui est un peptide de di-Ser(33)-exendine et/ou un peptide de di-Ala(35)-exendine et qui diffère du produit peptidique souhaité par un bloc constitutif de dimère d'acides aminés de di-sérine et/ou di-alanine, ladite impureté ne pouvant pas être séparée du produit peptidique ou d'un autre composant de la composition par une procédure chromatographique et ladite impureté étant coéluée ou sensiblement coéluée avec le produit peptidique souhaité après la procédure de séparation chromatographique,
    (b) fourniture d'au moins un échantillon de la composition de produit peptidique sans ajout de ladite impureté et d'au moins un échantillon supplémentaire de la composition de produit peptidique avec ajout d'une quantité connue de ladite impureté,
    (c) détermination quantitative de ladite impureté dans ledit échantillon de l'étape (b) par spectrométrie de masse, et
    (d) calcul de la quantité de ladite impureté dans la composition de produit peptidique sur la base des résultats de (c).

2. Procédé de détermination quantitative d'une impureté présente dans une composition de produit peptidique, comprenant les étapes de :

    (a) fourniture d'une composition de produit peptidique comprenant un produit peptidique, qui est un produit peptidique d'exendine comprenant la séquence d'acides aminés S-S-G-A et une quantité inconnue d'au moins une impureté qui est un peptide de di-Ser(33)-exendine et/ou un peptide de di-Ala(35)-exendine et qui diffère du produit peptidique souhaité par un bloc constitutif de dimère d'acides aminés de di-sérine et/ou di-alanine, ladite impureté ne pouvant pas être séparée du produit peptidique ou d'un autre composant de la composition par une procédure chromatographique,
    (b) fourniture d'au moins trois échantillons de la composition de produit peptidique, un premier échantillon comprenant la composition de produit peptidique sans ajout de ladite impureté, et au moins deux échantillons supplémentaires comprenant la composition de produit peptidique, chacun avec ajout d'une quantité connue différente de ladite impureté,
    (c) détermination quantitative de ladite impureté dans lesdits échantillons de l'étape (b) par spectrométrie de masse, et
    (d) calcul de la quantité de ladite impureté dans la composition de produit peptidique sur la base des résultats de (c).

3. Procédé selon la revendication 1 ou 2, dans lequel le produit peptidique a été chimiquement synthétisé, en particulier par une procédure de synthèse en phase solide, ou produit par des processus d'ADN recombinant.

4. Procédé selon l'une quelconque des revendications 1 à 3, dans lequel la composition de produit peptidique est une formulation pharmaceutique ou une composition destinée à la fabrication d'une formulation pharmaceutique.

5. Procédé selon l'une quelconque des revendications 1 à 4, dans lequel le produit peptidique d'exendine est le lixisénatide (AVE0010).

6. Procédé selon l'une quelconque des revendications 1 à 5, dans lequel le produit peptidique comprend des quantités inconnues d'au moins 2 impuretés qui ne peuvent pas être quantitativement séparées du produit peptidique ou d'un autre composant de la composition par une procédure chromatographique.

7. Procédé selon l'une quelconque des revendications 1 à 6, dans lequel l'impureté est ajoutée à au moins un échantillon de composition de produit peptidique d'une préparation mère, de préférence d'au moins deux préparations mères comprenant des concentrations différentes de l'impureté.

8. Procédé selon l'une quelconque des revendications 1 à 7, dans lequel au moins 3 ou 4 échantillons supplémentaires avec différentes quantités connues d'impureté ajouté sont fournis et soumis à détermination par spectrométrie de masse.

9. Procédé selon l'une quelconque des revendications 1 à 8, dans lequel la spectrométrie de masse est une spectrométrie de masse à haute résolution, par exemple comprenant la spectrométrie de masse à transformée de Fourier.

10. Procédé selon l'une quelconque des revendications 1 à 9, dans lequel le calcul comprend une analyse par régression linéaire.

11. Procédé selon la revendication 10, dans lequel le calcul est effectué selon l'équation :

$$y = ax+b$$

dans laquelle

a = pente
y = aire de pic déterminée de l'impureté dans un échantillon
x = quantité de l'impureté ajoutée dans un échantillon b = intersection
et la quantité inconnue de l'impureté $x_t$ est obtenue comme suit :

$$x_t = b \cdot a^{-1}$$

12. Kit de réactifs pour déterminer la quantité d'impuretés dans une composition de produit de lixisénatide (AVE 0010), comprenant :

(i) au moins une préparation mère de di-Ser(33)-AVE0010 (SEQ ID NO: 2) H-G-E-G-T-F-T-S-D-L-S-K-Q-M-E-E-E-A-V-R-L-F-I-E-W-L-K-N-G-G-P-S-S-S-G-A-P-P-S-K-K-K-K-K-K-NH$_2$
et/ou
(ii) au moins une préparation mère de di-Ala(35)-AVE0010 (SEQ ID NO: 3) H-G-E-G-T-F-T-S-D-L-S-K-Q-M-E-E-E-A-V-R-L-F-I-E-W-L-K-N-G-G-P-S-S-G-A-A-P-P-S-K-K-K-K-K-K-NH$_2$.

13. Procédé de contrôle de qualité d'une composition comprenant un produit peptidique d'exendine comprenant la séquence d'acides aminés S-S-G-A, comprenant la détermination quantitative selon le procédé selon l'une quelconque des revendications 1 à 11 de la quantité d'au moins une impureté qui est un peptide de di-Ser(33)-exendine et/ou un peptide de di-Ala(35)-exendine et qui diffère du produit peptidique souhaité par un bloc constitutif d'acide aminé dimère de di-sérine et/ou di-alanine dans ladite composition.

14. Procédé de contrôle de qualité d'une composition comprenant un produit de lixisénatide (AVE0010), comprenant la détermination quantitative selon le procédé selon l'une quelconque des revendications 1 à 11 de la quantité d'au moins une impureté qui est un di-Ser(33)-AVE0010 et/ou un di-Ala(35)-AVE0010 et qui diffère du produit peptidique souhaité par un bloc constitutif d'acide aminé dimère de di-sérine et/ou di-alanine dans ladite composition.

Figure 1

Figure 2

Figure 3

EP 2 841 950 B1

Figure 4

Figure 5

EP 2 841 950 B1

Figure 6

EP 2 841 950 B1

RT: 11.00 - 15.00          SM: 7G

RT: 11.00 - 15.00          SM: 7G

RT: 12.01
AA: 1818085

1237.15286
R=78713

Di-Ser (33)-AVE0010

1237.12496
R=69257

RT: 11.00 - 15.00         SM:   7G

RT: 12.12
AA: 1491701

4928.59084

Di-Ala (35)-AVE0010

RT: 11.00 - 15.00                   SM:    7G

RT: 12.01

AA: 374748

4944.58219

Di-Ser (33)-AVE0010

4944.46934

Figure 9

Di-Ala(35)-AVE0010 Standard addition - 1st prep.
Standard additions method on transformed data (lambda = 0.00)

Y=2.637E+06 + 6.190E+06×X
90% CI for individual values

Estimated amount [90% CI] (%%): 0.4260 [0.3834 ; 0.4687]

EP 2 841 950 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012012352 A2

**Non-patent literature cited in the description**

- **LEIB et al.** Direct Quantitation of Peptide Mixtures without Standards Using Clusters Formed by Electrospray Ionization Mass Spectrometry. *ANALYTICAL CHEMISTRY,* vol. 81 (10), 3965-3972